# EUROPEAN PATENT SPECIFICATION

(11) **EP 0 702 713 B1**
(45) Date of publication and mention of the grant of the patent: **30.01.2002**
(21) Application number: 94903334.4
(22) Date of filing: 30.11.1993
(51) Int. Cl.: C11D 11/00, C11D 3/386

(54) **ENZYMATIC METHODS AND USE OF ENZYMES FOR PRODUCING STONEWASHED LOOK ON INDIGO-DYED DENIM FABRIC**
ENZYMATISCHE VERFAHREN UND VERWENDUNG VON ENZYMEN ZUR HERSTELLUNG VON STONE WASH AUSSEHEN AUF INDIGO GEFÄRBTEM GEWEBE
PROCEDES ENZYMATIQUES DE PRODUCTION DE CROISE DE COTON TEINT EN INDIGO PRESENTANT L'ASPECT D'UN TISSU LAVE A LA PIERRE ET USAGE D'ENZYMES A CET EFFET

(30) Priority: 11.06.1993 US 75657
(43) Date of publication of application: 27.03.1996
(73) Proprietor: GENENCOR INTERNATIONAL, INC., Palo Alto, California 94304-1013 (US)
(72) Inventor: CLARKSON, Kathleen, A., San Francisco, CA 94110 (US); LAD, Pushkaraj, J., San Mateo, CA 94403 (US); MULLINS, Margaret, M., Montara, CA 94037 (US); SIMPSON, Curran, M., Montara, CA 94037 (US); WEISS, Geoffrey, L., San Francisco, CA 94117 (US); JACOBS, Lindsay, San Mateo, CA 94402 (US)
(74) Representative: Kiddle, Simon John
(86) International application number: US9311555
(87) International publication number: WO9429426

(56) References cited:
- WO-A-90/07569
- WO-A-91/05841
- WO-A-92/06183
- WO-A-94/03577
- GB-A- 2 094 826
- US-A- 4 566 985
- US-A- 4 760 025
- AMERICAN DYESTUFF REPORTER, vol.79, no.9, September 1990, USA pages 24 - 28 KOCHAVI, VIDEBAEK, CEDRONI 'Optimizing Processing Conditions in Enzymatic Stonewashing' cited in the application

## Description

### Field of the Invention:

This invention relates to methods for reducing or preventing the backstaining of blue indigo dye onto denim during the stonewashing of denim fabric and garments utilizing cellulase enzymes.

### Background of the Invention:

Denim is cotton cloth which has been dyed, usually blue, with the dye indigo. One desirable characteristic of indigo-dyed denim cloth is the alteration of dyed threads with white threads, which upon normal wear and tear gives denim a white on blue appearance. A popular look for denim is the stonewashed look. Traditionally stonewashing has been performed by laundering the denim material in the presence of pumice stone which results in fabric having a faded or worn appearance with the desired white on blue contrast appearance described above. This stonewashed look primarily consists of removal of dye in a manner to yield a material with areas which are lighter in color, while maintaining the desirable white on blue contrast, and a material which is softer in texture.

Enzymes, particularly cellulases, are currently used in processing denim. In particular cellulases have been used as a replacement for or in combination with pumice stones for the traditional "stonewashing" process used to give denim a faded look. Use of enzymes to stonewash has become increasingly popular because use of stones alone has several disadvantages. For example, stones used in the process cause wear and tear on the machinery, they cause environmental waste problems due to the grit produced and result in high labor costs associated with the manual removal of the stones from pockets of garments. Consequently, reduction or elimination of stones in the wash may be desirable.

Contrary to the use of pumice stones, enzymes (particularly cellulases) are safe for the machine, result in little or no waste problem and drastically reduce labor costs.
Therefore, it may be beneficial to use enzymes for stonewashing. However, even though the use of enzymes such as cellulase may be beneficial as compared to stones alone, there are some problems associated with the use of enzymes for this purpose. For example, one problem with some cellulases, such as cellulases from *Trichoderma,* is what could be described as an incomplete removal of dye caused by "redeposition" or "backstaining" (both terms used interchangeably herein) of some of the dye back onto the fabric during the enzymatic stonewashing process. Such redeposition or backstaining causes blue coloration of the surface, resulting in less contrast between the blue and white threads and abrasion points (i.e., a blue on blue look rather than the preferred white on blue). See American Dyestuff Reporter, Sept. 1990, pp. 24-28.
Redeposition or backstaining is objectionable to some users. Even though *Trichoderma* cellulases exhibit backstaining, they are preferable to *Humicola* cellulases, which do not generally exhibit backstaining, because of the higher specific activity on denim material seen with *Trichoderma* cellulases. In addition, cellulases with a higher degree of purity may be beneficial in the present invention. High specific activity or a high level of purity may result in a higher degree of abrasion in significantly shorter processing times and, therefore, is preferable to the denim processors.

The problem of redeposition of dye during stonewashing has been a concern of denim processors. Previous attempts to address the problem include addition of extra antiredeposition chemicals, such as surfactants or other agents, into the cellulase wash to help disperse the loosened indigo dye and reduce redeposition. In addition, denim processors have tried using cellulases with less specific activity on denim, along with extra rinsings. This results in additional chemical costs and longer processing times. Another method attempting to address the redeposition problem includes adding a mild bleaching agent or stain removing agent in the process. This method affects the final shade of the garment and increases processing time.

US Patent No. 4,566,985 discloses an enzyme cleaning composition containing a mixture of enzymes including a protease and benzamidine hydrohalide.

WO-A-94/03577, available as intervening prior art under Art 54(3)EPC, discloses an enzyme containing washing agent including cellulase and a proteolytic agent in addition to other constituents.

WO-A-90/07569 discloses the use of cellulose for stonewashing denim. The cellulase component can also include pumice stones or amylases. After the stonewashing step is complete, the reference discloses that the cellulase can be eliminated using extremes of temperature or pH, using detergents or using a protease.

GB 2094826A discloses detergent compositions comprising surfactant and cellulase. In one example, the compositions additionally include lipases, amylases and/or proteases.

While these methods aid to some limited degree in the reduction of redeposition, the methods are not entirely satisfactory and some objectionable backstaining remains. Use of enzymes and stones together may be advantageous in overcoming this redeposition problem; however, it leave the processor with some of the problems associated with the use of stones alone.

Based on the shortcoming of previously attempted methods for reducing or prevent redeposition, there is a need for more environmentally favorable and more cost effective methods to address the issue of redeposition or backstaining of dye during stonewash treatment.

Accordingly, it would be desirable to find a method that would enhance the removal of the dye during stonewashing when redepositing or backstaining cellulases are used which, although exhibiting backstaining, have a high degree of specific activity on denim material.

### Figures:

Figure 1 shows graphics of comparative reflectance measurements of stone washing with a redepositing cellulase and different added proteases, as described in Examples 1, 2 and 3, vs. a redepositing cellulase control.

Figure 2 shows graphics of comparative reflectance measurement of stonewashing with a redepositing cellulase and different added proteases vs. a redepositing cellulase control. As exemplified in Example 4.

### Summary of the Invention:

In one aspect, the present invention provides a method for introducing into the surface of indigo-dyed denim, localized areas of variation in color density and a high contrast between blue and white fibers of the denim, characterised in that said method comprises contacting the denim simultaneously with:
a. an effective amount of a redepositing cellulase sufficient to produce the variations in color density; and,
b. an effective amount of an added protease sufficient to reduce backstaining and increase the contrast between blue and white fibers;
with the proviso that the cellulase is not an enzyme preparation of penicillum funiculosum containing cellulases and anylases.

In a further aspect, the present invention provides the use of a protease and a redepositing cellulase for reducing backstaining when the cellulase is used to introduce localized areas of variation of color density into the surface of indigo-dyed denim, thereby producing a high contrast between blue and white fibers of the denim.

Applicants have found that treatment of cotton indigo-dyed denim with an effective amount of a redepositing or backstain inhibiting composition comprising a redepositing cellulase composition and added protease that is in excess of the amounts naturally present in a redepositing cellulase preparation produced by a natural organism is an improvement over redepositing cellulase alone as a method for introducing variations in color density into the surface of the indigo-dyed denim. The result of treatment with such a composition is an improvement in the contrast between white and blue threads, achieving more complete dye removal (more like that achieved with pumice stones). The improvement in the contrast is due to a reduction in dye redeposition, resulting in abrasion points which are more distinct and display greater contrast between white and blue yarns, giving a superior stonewashed look. A small percentage of surface active chemical surfactant may optionally be added to the compositions or methods described herein. If a surface active agent is added, it may be added either with the cellulase and protease in the wash or with the protease as an after treatment rinse to enhance the proteolytic effect.

### Detailed Description of the Invention:

Denim that is stonewashed with the addition of an effective amount of added protease during cellulase treatment with a redepositing cellulase shows a dramatic reduction in the level of backstaining and a visible increase in the contrast between white and blue threads, i.e., a more complete stonewash effect. While applicant does not wish to be held by any particular theory, one possible explanation for this might be that certain components present in redepositing cellulase compositions (see definition below) which comprise mixtures of several enzymes may bind tightly to the denim surface. These components may also bind dye-stained cellulose fragments and/or the dye itself, thus binding the colored materials or dye back on to the fabric. The addition of added protease effectively removes or prevents the cellulase proteins from binding the colored particles back onto the surface of the denim and yet, surprisingly, does not adversely effect the resultant abraded look caused by the action of the cellulase.

Prior to discussing this invention in further detail, the following terms will be defined.

The term "added protease" refers to an incremental amount of protease over the amount which is produced naturally by a microorganism. This incremental amount will result in reduction of backstaining when added to a redepositing cellulase composition during the stonewashing process.

Preferably, such an incremental amount is at least 1% more than the amount of total protease protein naturally occurring in the microorganism that produces the redepositing cellulase composition. The amount of added protease is determined by many factors such as the purity, activity and specificity of the added protease, for example. Therefore, the incremental amount of added protease will vary with the type of protease added to the redepositing cellulase composition.

"Added protease", as used herein, may be derived from either exogenous or endogenous sources. Added exogenous protease refers to those proteases which degrade proteins that are derived or developed externally from those naturally present in the cellulase composition of the microorganism.
Alternatively, the added protease may be endogenous. In this case, added endogenous proteases refers to an amount of protease in a redepositing cellulase composition that is over and above what is naturally produced by the microorganism by overexpression of the gene encoding the naturally occurring protease. The amount of enhanced protease produced by overexpressing the gene encoding the protease is an amount that will result in inhibiting or reducing backstaining in the stonewashing process.
Preferably, the enhancement is at least 1% greater than the amount of protease naturally occurring in the microorganism.

Proteases are available from several sources including microbial, plant and animal sources and are well documented in the literature. Some important commercial proteolytic sources include *Bacillus licheniformis, Bacillus subtilis* and *Aspergillus oryzae*. Proteases suitable for the invention include, for example, serine, metallo and acid proteases, as well as endo- and exo-proteases. Subtilisins are serine proteases which generally act to cleave internal peptide bonds of proteins and peptides. Metallo proteases are exo- or endo-proteases which require a metal cofactor for activity. One of the preferred serine proteases is subtilisin. Particularly preferred proteases useful in the present invention are proteases obtained from a microorganism genetically modified as described in US Patents 4,760,025 and 5,185,258.

The term "cellulase composition" comprises one or more exocellobiohydrolase (CBH), endogluconase (EG) and β-glucosidase (BG) components produced by a naturally occurring microorganism, wherein each of these components is found at the ratio naturally produced by the microorganism and sometimes referred to herein as a "complete or natural cellulase composition."

It is contemplated that the cellulase compositions used in the method of the present invention may also include a cellulase composition obtained from a microorganism genetically modified so as to overproduce, underproduce or not produce one or more of the CBH, EG and/or BG components of cellulase. Additional modified cellulase compositions may include truncated cellulase proteins comprising either the binding domain or the core domain of the CBHs or EGs, or a portion or derivative thereof. Other examples of modified cellulase compositions may include alterations in the degree of glycosylation, or substitution(s) of amino acid(s) of the primary sequence of the cellulases or truncated cellulases.

A "redepositing or backstaining cellulase" as used herein refers to those cellulases, for example *Trichoderma*, which in the enzymatic stonewashing of denim tend to backstain the fabric leading to incomplete stonewashing when compared with stones alone or to cellulases which do not backstain, such as from *Humicola*. Redepositing or backstaining cellulases are derived from microorganisms such as fungal microorganism *Trichoderma sp.* or any other microorganism that produce cellulases displaying a backstaining or redepositing effect on denim in the stonewashing process.

The methods of the present invention comprise contacting the denim to be partially or wholly enzymatically stonewashed with an added protease in an amount sufficient to reduce backstaining and thus, to increase the contrast between blue and white fibers. The protease may be mixed together with the cellulase and then added to the wash containing the indigo-dyed denim fabric or each can be added separately and directly to the wash or the protease may be added subsequent to the cellulase wash in the rinse cycle provided said cellulase is not enzyme preparation of Penicillium funicolosum containing cellulases and amylases. Alternatively, the protease may be added to the wash cycle prior to the addition of the cellulase to the same wash cycle. It should be noted that the cellulase stonewash bath may or may not include the liquor from the previous desizing step.

The redepositing or backstain inhibiting compositions used in the present invention comprise added protease(s) and redepositing cellulase(s). In a particular embodiment, the redepositing or backstain inhibiting composition used in the present invention comprises a redepositing or backstaining cellulase and added protease in a ratio of from about 5 ppm total protein in the redepositing cellulase: 1,000 ppm total protein in the added protease to 1,000 ppm total protein in the redepositing cellulase: 1 ppm total protein in the added protease. In a more preferred embodiment, the ratio is from about 10 ppm total protein in the redepositing cellulase: 200 ppm total protein in the added protease to 200 ppm total protein in the redepositing cellulase: 1 ppm total protein in the added protease. Total cellulase and protease protein can be measured by various assay methods known in the art. The assay preferably used herein is a commercially available biuret Lowry assay sold by Sigma Company, using bovine serum albumin (BSA) as the reference standard.

The redepositing or backstain inhibiting compositions may further comprise various adjuvants as known to those skilled in the art. For example, a surfactant (anionic or nonionic) compatible with the redepositing cellulase and added protease would be useful in such compositions. Preferable surfactants are nonionic, such as the polyoxyethylated alcohols found in the TRITON® series of surfactants (octylphenoxypolyethoxyethanol nonionic surfactants) which are commercially available from Union Carbide. See, for example, US Patent 5,006,126 for a sample of these compatible surfactants. It should be noted that inclusion of a surfactant may further increase the stonewashing effect in combination with the redepositing cellulase and added protease. Other materials can also be used with or placed in the composition as desired, including stones, fillers, solvents, buffers, pH control agents, enzyme activators, builders, enzyme stabilizers and other antiredeposition agents.

The backstain inhibiting composition may be formulated as a solid product wherein the solid may be granular, spray dried or agglomerated. For example, enzyme containing granules wherein the layer may comprise one or more enzymes including cellulases, proteases, amylases, and other proteins as recited in WO 93/07263. One contemplated application for a particle coated with cellulase, protease and amylase is to combine the desizing and stonewashing treatment in a single wash cycle.

Alternatively, the backstain inhibiting compositions may be formulated as a liquid gel or paste product. In this particular embodiment, redepositing cellulase is mixed with added protease in a ratio of from about 5 ppm total protein in the redepositing cellulase: 1,000 ppm total protein in the added protease to 1,000 ppm total protein in the redepositing cellulase: 1 ppm total protein in the added protease. To prepare a stable aqueous preparation of the above mixture, stabilizing ingredients must also be adding comprising oxygen containing, water soluble organic solvents in a buffer ranging from pH 4-6. Preferably sorbitol and glycerol are the stabilizing ingredients of choice.

In a preferred embodiment, the above liquid formulation mixture is incubated at elevated temperatures ranging from about 30°C to about 60°C from about 1 hour to 2 weeks prior to application on the denim fabric. In the most preferred embodiment, a temperature of about 37°C and incubation time of about 120 hours are employed. One skilled in the art will recognize the amount of time for incubation which depends on the temperature chosen to prepare the mixture. One skilled in the art will further recognize that lower temperatures may be employed; however, longer reaction times may be required depending on the cellulases and proteases utilized in the composition. It is further contemplated that the pretreated liquid formulation may be converted to a solid, i.e. granular form, to preserve the stability of the composition.

Added proteases used in the present invention may be added together with the redepositing cellulase to the stonewashing bath, or each added separately to the stonewash cellulase bath. Alternatively, the protease may be added in a separate cycle either prior to the cellulase bath or in a subsequent rinse treatment solution provided the cellulase is not an enzyme preparation of Penicilium funicolosum containing cellulases and amylases. In all methods contemplated above, the redeposition is reduced by about 5%, preferably about 10%, via measurement of reflectance off the backside of treated garments when compared to the stonewashing with cellulase alone. The measurement of reflectance values is described more fully in the Experimental section of this application. It should be noted, however, that the values measured for reflectance are compressed as compared to the visual differences seen when comparing treated versus untreated fabric. Visual observation is a more sensitive indicator of the stonewashing effect. Thus, visual observation of the backstaining on the backside of the garment reveals greater differences between treated and untreated fabric, as compared to measurements determined by a reflectometer.

In an embodiment of the present invention, the redepositing cellulase and added protease are present in a ratio of from about 5 ppm total protein in the redepositing cellulase: 1,000 ppm total protein in the added protease to 1,000 ppm total protein in the redepositing cellulase: 1 ppm total protein in the added protease. In a more preferred embodiment, the ratio is from about 10 ppm total protein in the redepositing cellulase: 200 ppm total protein in the added protease to 200 ppm total protein in the redepositing cellulase: 1 ppm total protein in the added protease.

One skilled in the art will realize that the effective amount of added protease will vary depending upon a number of well understood parameters, including the amount and purity of redepositing cellulase used, as well as the amount of redeposition which occurs without protease, the contact time, the amount of dye removed during stonewashing, the specific activity of the redepositing cellulase and/or added protease, the pH and temperature of the stonewashing process, the formulation of the product (liquid versus granular) and the like. It is well known in the art that specific activity of added protease and/or redepositing cellulase can be modified by genetically engineering a strain to change or modify components of interest. For example, the overexpression of certain cellulase components is demonstrated in WO 92/17572 and WO 92/17574. Protein engineering techniques can also be used to modify enzyme activity or specificity, see for example US Patent 4,760,025.

It will be a simple matter to titrate the added protease with several washings and visually observe the resultant denim samples to achieve an effective amount which results in a reduction in backstaining. It should be noted, however, that there is a balance between the proteolytic effect on reducing backstaining and the proteolytic effect on reducing abrasion. One must find the optimum ratio of redepositing cellulase to added protease to achieve the antiredeposition effect without adversely affecting abrasion.

Reflectance values can be used as well to track the degree of redeposition on the backside of the garment but do not accurately reflect the contrast between fibers on the abraded front side of the garments. Differences in redeposition determined visually are more pronounced than with reflectance values, but reflectance values do show the effect to a lesser extent.

Redepositing or backstain inhibiting compositions of the present invention for addition to denim stonewash solutions (either as a solid or liquid) while comprising a redepositing cellulase(s) and added protease(s), may further comprise other adjuvants, such as surfactants, fillers, dispersants, buffers or pH control agents, enzyme activators, builders, enzyme stabilizers or other antiredeposition ingredients. One skilled in the art can readily compare the results of various combinations and ratios of solutions to optimize the selected components of such compositions. However, applicants have found that depending on the type of enzymes used and the particular mixture of selected ingredients mentioned above, the range of enzyme ingredients will preferably be within a ratio of from about 5 ppm total protein in the redepositing cellulase: 1,000 ppm total protein in the added protease to 1,000 ppm total protein in the redepositing cellulase: 1 ppm total protein in the added protease, as previously defined. In a more preferred embodiment, the ratio is from about 10 ppm total protein in the redepositing cellulase: 200 ppm total protein in the added protease to 200 ppm total protein in the redepositing cellulase: 1 ppm total protein in the added protease. This ratio will accommodate various combinations of specific activity of both redepositing cellulase and added protease, from both high specific activity to both low to variations in between, where one enzyme is relatively high in specific activity and the other relatively low. In addition, this ratio will accommodate various combinations of different purity of enzymes, from both highly pure to both having low purity to variations in between where one enzyme is relatively pure and the other has relatively low purity.

Where a surfactant is included in the composition, it will be about 5-85% of the total weight of either the liquid or dry composition. However, based on the Examples, one skilled in the art may lower the concentration of surfactant to amounts below 5% of the total weight of the liquid or dry composition without departing from the scope of the present invention. It is also possible to add the components separately, all at once, or sequentially (including separate rinse cycles). The amount of composition to use to treat denim would depend on the amount of enzymes active on the denim substrate and their specific activity on that substrate, the desired amount of stonewash effect and other parameters within the skill in the art.

The following examples are illustrative of the effectiveness of the compositions and processes of the present invention and are not intended to be limiting. Other choices of added protease or redepositing cellulase, as well as wash conditions such as concentration, measurement, pH, temperature and the like, will be evident to those skilled in the art based on the teachings herein.

### Experimental:

### EXAMPLE 1

A 50 lb. (23 kg) Unimac dye/washing machine was used. Approximately 10 lbs. (~3.8 kgs.) of desized test denim garments were placed in the machine. The machine was filled with 10 gals. (38 L) hot water and brought to 131ºF (55ºC). The liquor ratio was 10:1 (kg. garment:liters liquor). The liquor was buffered to Ph ∼4.9 with 44 grams citric acid, monohydrate, and 100 grams sodium phosphate dibasic.

Once pH was established, redepositing cellulase enzyme INDIAGE® 44L (*Trichoderma* cellulase composition, commercially available from Genencor International, Inc.) was added at a rate of about 0.5 ml of product/L of wash liquor (62.5 ppm total protein). Protease enzyme, GC899 (a serine endopeptidase from *Bacillus subtilis*, available from Genencor International, Inc.) was then added at about 2.5 ml of product/L of wash liquor (163 ppm total protein). This resulted in a dose ratio of about 1:2.6 based on total protein. The garments were washed at 36 rpms for 60 minutes. After this, the bath was dropped.

The garments were then rinsed according to a standardized protocol in three consecutive cycles of clean liquor. Rinse #1 = 24 gals. (91 L) hot water, approximately 50ºC, plus ∼100 grams standard detergent WOB (from American Association of Textile Chemists and Colorists [AATCC], WOB = without brighteners). Agitation was for 12 minutes at 36 rpms. The bath was dropped. Rinse #2 = 24 gals. (91 L) warm water, ~40ºC, with no additional detergents, agitated for 5 minutes. The bath was dropped. Rinse #3 = 24 gals. (91 L) cold water, ∼30ºC, with no additional detergents, agitated for 5 minutes. The bath was dropped. Garments were extracted and dried in a standard electric clothes dryer.

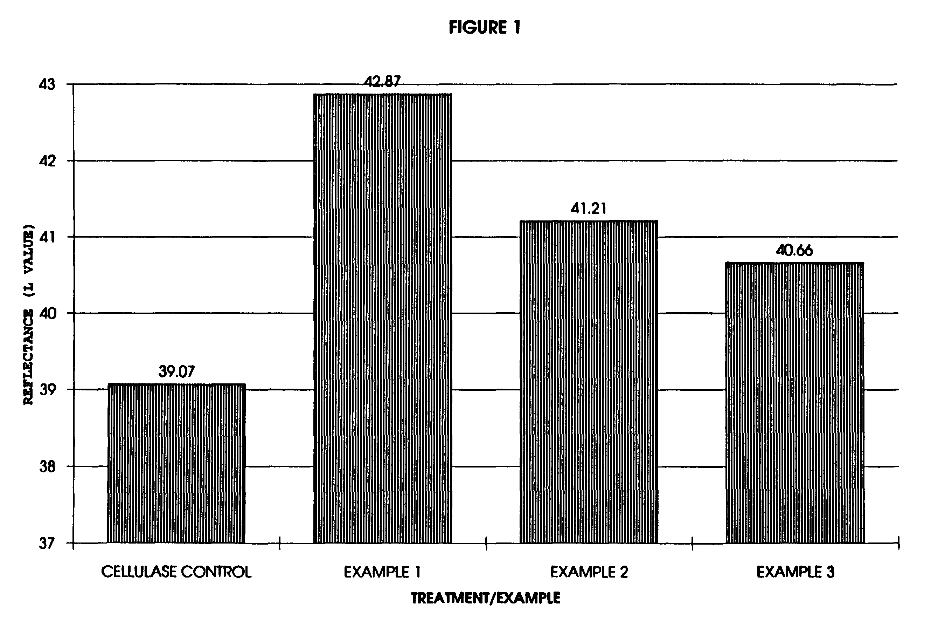

Reflectance readings were taken off the backside of the garments using a Hunter Color Difference Meter (reflectometer apparatus). Reflectance was measured as the percent reflectance (or transmittance of light off treated fabric) where L = 100 units is white, and L = 0 units is black. Compared to redepositing cellulase treatment only (redepositing cellulase control = 0.5 ml of INDIAGE® 44L product/L wash liquor), the redepositing cellulase plus added protease treated garments resulted in significantly reduced backstaining with similar levels of abrasion (39.07 [redepositing cellulase control] vs. 42.87 [redepositing cellulase and added protease] reflectance values). These reflectance values confirmed visual observations. The added protease treatment resulted in a better abraded contrast overall. See Figure 1.

### EXAMPLE 2

This test was substantially similar to Example 1 with the same type and amount of redepositing cellulase being used but with a different amount of the same added protease. This was about 0.5 ml of product/L of wash liquor (33 ppm total protein) of the added protease. This resulted in a dose ratio of about 1:0.5 based on total protein. All other processing parameters were the same.

Compared to redepositing cellulase control, reflectance readings were significantly different between the two treatments, but level of abrasion was not. Cellulase control = 39.07 vs. cellulase and protease = 41.21. The final abraded look had better contrast for the added protease treated garments as compared to untreated (no protease) control, however, added protease treatment in Example 1 was better than Example 2, showing the titration effect of added protease. See Figure 1.

### EXAMPLE 3

The redepositing cellulase treatment was the same as in Example 1 but without the addition of added protease. The bath was dropped and the standard rinse cycle was begun, as described in Example 1 with the following exception: 1 ml of GC899 protease per liter of rinse liquor was added at the beginning of Rinse #1. A total of 100 mls of added protease product was used (65 ppm total protein). All other conditions remained the same.

Use of the added protease with detergent in the rinse cycle resulted in significantly reduced backstaining when compared to cellulase control rinsed with detergent alone.
Reflectance values were 40.66 for the added protease rinse vs. 39.07 for the standard rinse without added protease. The degree of abrasion was the same for both treatments, although the added protease rinsed garments showed better overall contrast than the standard rinse without added protease. See Figure 1.

### EXAMPLE 4

Utilizing the cellulase washing protocol described in Example 1, the following added protease products were tested, along with 0.5 ml of product/L of wash liquor of redepositing cellulase enzyme (62.5 ppm total protein) INDIAGE® 44L *(Trichoderma* cellulase composition, commercially available from Genencor International, Inc.):
1. MULTIFECT™ P64 (bacterial protease derived from *Bacillus licheniformis* and commercially available from Genencor International, Inc.), dosed at about 5 g of product/L of wash liquor (71 ppm total protein) to yield a dose ratio of about 1:1 based on total protein;
2. MULTIFECT™ P53 (bacterial protease derived from *Bacillus subtilis* and commercially available from Genencor International, Inc.), dosed at about 5 g of product/L of wash liquor (88 ppm total protein) to yield a dose ratio of about 1:1.5 based on total protein;
3. MULTIFECT™ P41 (fungal protease derived from *Aspergillus oryzae* and commercially available from Genencor International, Inc.), dosed at about 5 g of product/L of wash liquor (172 ppm total protein) to yield a dose ratio of about 1:2.75 based on total protein;
4. Subtilisin GC399 (available from Genencor International, Inc.), dosed at about 2.5 g of product/L of wash liquor (238 ppm total protein) to yield a dose ratio of about 1:4 based on total protein.

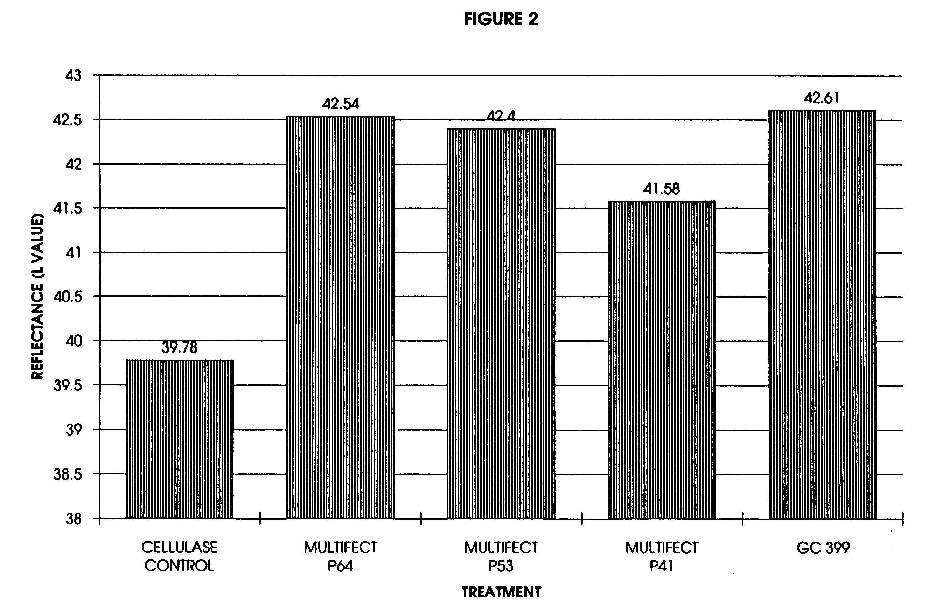

All added protease treatments resulted in less redeposition with similar abrasion levels when compared to redepositing cellulase control garments. In each case, added protease treatment improved overall contrast of the abraded look. See Figure 2. This example shows the effect of added proteases from various microbial sources which show the same antiredeposition effect as the added protease used in the previous examples.

### EXAMPLE 5

A series of cellulase washes were run in order to demonstrate the efficacy of added protease protein *per se* as opposed to the formulation components of the added protease product in reducing the degree of redeposition. GC899 protease protein was used, which contained no enzyme product formulation components other than the protease protein. The same cellulase washing procedure was used as described in Example 1.

The following treatments were run:
1. Buffer Control (no protease or cellulase).
2. Redepositing Cellulase Control = 0.5 ml of product/L wash liquor (62.5 ppm total protein) dose of INDIAGE® 44L *(Trichoderma* cellulase composition, commercially available from Genencor International, Inc.).
3. Nonredepositing Cellulase Control = 2.5 ml of product/L wash liquor (100 ppm total protein) dose of DENIMAX™ L (endoglucanase derived from *Humicola,* a non-pathogenic mold and commercially available from Novo Nordisk). This was the recommended dose of the manufacturer.
4. Added Protease Treatment = 0.5 ml of product/L wash liquor (62.5 ppm total protein) dose of INDIAGE® 44L *(Trichoderma* cellulase composition, commercially available from Genencor International, Inc.) plus about 0.18 mls of GC899 protease protein/L wash liquor (25 ppm total protein). This resulted in a dose ratio of about 1:0.4 based on total protein.

All treatments were run at Ph 5 except for the nonredepositing cellulase treatment, which was run at Ph 7 according to the manufacturer's recommendations. The reflectance readings are shown in the following Table 1:

**TABLE I**

| **TREATMENT** | **REFLECTANCE (L VALUE)** |
|---|---|
| Buffer Control | 43.25 |
| Redepositing Cellulase Control | 37.92 |
| Nonredepositing Cellulase Control | 42.60 |
| Added Protease Treatment | 43.51 |

Reflectance results correspond with visual observations that the addition of protease protein reduces the degree of redeposition on garments. The protease treated garments have similar reflectance readings to a nonredepositing cellulase treatment. Quality of abraded contrast is improved with protease treatment as well over redepositing cellulase treatment.

### EXAMPLE 6

Use of added protease in combination with a surfactant, either added separately with the redepositing cellulase or added as a redeposit inhibiting composition is demonstrated in the following treatments. Again, the same cellulase washing protocol was used for all cases, as described in Example 1.
1. Buffer Control (no protease or cellulase).
2. Redepositing Cellulase Control = 0.5 ml of product/L of wash liquor (100 ppm total protein) dose of a redepositing cellulase, CELLUSOFT™L *(Trichoderma* cellulase preparation, commercially available from Novo Nordisk), plus 0.25 ml of product/L of wash liquor (250 ppm) dose of nonionic surfactant, TRITON® X-100 (octylphenoxypolyethoxyethanol nonionic surfactant, commercially available from Union Carbide Chemicals and Plastics Co., Inc.).
3. Added Protease/Surfactant Treatment = 0.5 ml of product/L of wash liquor (100 ppm total protein) dose of a redepositing cellulase, CELLUSOFT™L (*Trichoderma* cellulase preparation, commercially available from Novo Nordisk), plus 0.25 ml of product/L of wash liquor (250 ppm) dose of nonionic surfactant, TRITON® X-100 (octylphenoxypolyethoxyethanol nonionic surfactant, commercially available from Union Carbide), plus about 0.2 mls of GC899 protease protein/L wash liquor (40 ppm total protein). this resulted in a dose ratio of about 1:0.4 based on total protein.
4. Nonredepositing Cellulase Control = 2.5 ml of product/L of wash liquor (100 ppm total protein) dose of DENIMAX™ L (endoglucanase derived from *Humicola,* a non-pathogenic mold and commercially available from Novo Nordisk).
5. Backstain Inhibiting Composition Blend = a blend comprising of redepositing *Trichoderma* cellulase (from Genencor International, Inc.), subtilisin protease (GC399 from Genencor International, Inc.) and nonionic surfactant (TRITON® X-120, octylphenoxypolyethoxyethanol nonionic surfactant, commercially available from Union Carbide) was dosed at 2 g of blend/L of wash liquor. This is about a 1:0.4 ratio of cellulase to protease protein as defined previously. This dose of the blended product resulted in doses of 60 ppm total protein from the cellulase product, 24 ppm total protein from the protease product and 120 ppm surfactant. The blend was made up of 3% total protein from the cellulase, 1.2% total protein from the protease and 6% of the surfactant.

**TABLE II**

| **TREATMENT** | **REFLECTANCE (L VALUE)** |
|---|---|
| Buffer Control | 43.25 |
| Redepositing Cellulase Control | 35.65 |
| Protease/Surfactant Treatment | 42.48 |
| Nonredepositing Cellulase Control | 42.60 |
| Backstain Inhibiting Composition Blend | 42.33 |

As can be seen, the addition of protease to a redepositing cellulase in the presence of surfactant, either as separate components added altogether (protease/surfactant treatment), or as a single backstain inhibiting composition (backstain inhibiting composition blend), markedly reduces the degree of redeposition. Reflectance values of protease treatments are similar to those of a nonredepositing cellulase or buffer treatment. Visual observations confirm this. The contrast of the abraded look is also improved with protease treatment which is better than treatment with the redepositing cellulase control.

### EXAMPLE 7

The test was performed in a substantially similar manner as Example 1, except that the protease and redepositing cellulase are mixed together prior to the addition in the wash cycle. The mixture, INDIAGE 44L and Protease GC899 is prepared in a ratio of about 10:1 based on total protein. In this example, the amount is about 111 ppm total protein. Once the pH of the washing liquid was established, the redepositing cellulase and protease mixture was added at a concentration of about 1.0 ml of mixture/L of wash liquid. All other processing parameters were the same as Example 1 except that a different lot of denim was employed in this Example and Example 8 below.

Compared to the redepositing cellulase control, reflectance readings of the sample treated with redepositing cellulase/protease were significantly different.
Reflectance results correspond with the visual observation that the addition of protease protein in the mixture reduces the degree of redeposition on the denim garments compared to the redepositing cellulase control. The protease treated garments show similar reflectance readings to a non-depositing cellulase treatment. The quality of abraded contrast in the fabric was also improved with the redepositing cellulase/protease mixture compared to the redepositing cellulase alone. See Table III below.

### EXAMPLE 8

The test was performed in a similar manner as Example 7 using the same redepositing cellulase and protease mixture, however, the ratio of cellulase to protease was changed and an additional heating process was included.

The mixture comprising INDIAGE 44L and Protease GC899 was prepared in a ratio of about 60:1 based on total protein. The mixture was further heated to a temperature of 37°C for 120 hours before addition to the wash cycle. In this example, the amount is about 122 ppm total protein. All other processing parameters were the same as those described in Examples 1 and 7.

**TABLE III**

| TREATMENT | REFLECTANCE (L VALUE) |
|---|---|
| Redepositing Cellulase Control | 45.08 |
| Redepositing Cellulase/Protease Mixture (Example 7) | 52.58 |
| Redepositing Cellulase/Protease Mixture Heat Treatment (Example 8) | 52.53 |
| Nonredepositing Cellulase Control | 51.86 |

Similar to results in Example 7 where garments are treated with a redepositing cellulase/protease mixture, treating garments with the redepositing cellulase/protease mixture that has been previously heated results in improved reduction of backstaining compared to the garments treated with redepositing cellulase alone. Moreover, the contrast of the abraded look is markedly improved using the preincubated cellulase/protease mixture compared to the redepositing cellulase control and the redepositing cellulase/protease mixture of Example 7.

## Claims

1. A method for introducing into the surface of indigo-dyed denim, localized areas of variation in color density and a high contrast between blue and white fibers of the denim, **characterised in that** said method comprises contacting the denim simultaneously with:
a. an effective amount of a redepositing cellulase sufficient to produce the variations in color density; and,
b. an effective amount of an added protease sufficient to reduce backstaining and increase the contrast between blue and white fibers;
with the proviso that the cellulase is not an enzyme preparation of Penicillium funiculosum contaning cellulases and amylases.

2. The method according to claim 1, wherein the effective amount of cellulase and protease is a ratio from about 5 ppm total redepositing cellulase protein: 1,000 ppm total added protease protein to 1,000 ppm total redepositing cellulase protein: 1 ppm total added protease protein.

3. The method according to claim 1 or claim 2, wherein said added protease is a serine or metallo protease.

4. The method according to any one of claims 1 to 3, wherein the protease is from *Bacillus* or *Aspergillus.*

5. The method according to any one of the preceding claims, further comprising the addition of a surfactant in an amount sufficient to further increase the removal of redeposited indigo dye.

6. The method according to any one of the preceding claims, wherein the redepositing cellulase is from *Trichoderma.*

7. The method according to any one of the preceding claims, wherein said added protease or said redepositing cellulase has been modified by genetic engineering to yield a strain which overexpresses particular components of the protease or cellulase or is deleted for specific components.

8. The method according to any one of the preceding claims, wherein said added protease or said redepositing cellulase has been modified by protein engineering to yield protease or cellulase enzymes having altered properties such as enzyme activity or specificity.

9. The method according to any one of the preceding claims, further comprising contacting the denim with pumice stones.

10. The method according to claim 3, wherein said serine protease is a subtilisin.

11. The method according to claim 1, wherein said redepositing cellulase and said protease are mixed together prior to contacting said denim and said mixture is maintained at a temperature ranging from about 30°C to about 60°C for about 1 hour to about 14 days.

12. Use of a protease and a redepositing cellulase for reducing backstaining when the cellulase is used to introduce localized areas of variation of color density into the surface of indigo-dyed denim, thereby producing a high contrast between blue and white fibers of the denim.

13. The use according to claim 12, wherein the cellulase and protease are used in a ratio from about 5 ppm total redepositing cellulase protein: 1,000 ppm total added protease protein to 1,000 ppm total redepositing cellulase protein: 1 ppm total added protease protein.

14. The use according to claim 12 or claim 13, wherein the protease is a serine or metallo protease.

15. The use according to any one of claims 12 to 14, wherein the protease is from *Bacillus* or *Aspergillus.*

16. The use according to any one of claims 12 to 15, further comprising contacting the denim with a surfactant in an amount sufficient to further increase the removal of redeposited indigo dye.

17. The use according to any one of claims 12 to 16, wherein the redepositing cellulase is from *Trichoderma.*

18. The use according to any one of claims 12 to 17 wherein the protease or the redepositing cellulase has been modified by genetic engineering to yield a strain which overexpresses particular components of the protease or cellulase or is deleted for specific components.

19. The use according to any one of claim 12 to 18, wherein the protease or the redepositing cellulase has been modified by protein engineering to yield protease or cellulase enzymes having altered properties such as enzyme activity or specificity.

20. The use according to any one of claims 12 to 19, further comprising contacting the denim with pumice stones.

21. The use according to claim 14, wherein the serine protease is a subtilisin.

22. The use according to any one of claims 12 to 21, wherein the redepositing cellulase and the protease are mixed together prior to contacting said denim and said mixture is maintained at a temperature ranging from about 30°C to about 60°C for about 1 hour to about 14 days.

## Patentansprüche

1. Verfahren zum Einführen lokaler Variationsbereiche bezüglich der Farbdichte in die Oberfläche von indigo-gefärbtem Denim sowie eines hohen Kontrasts zwischen blauen und weißen Fasern des Denims, **dadurch gekennzeichnet, dass** das Verfahren das Kontaktieren des Denims gleichzeitig mit folgendem umfasst:
a) einer wirksamen Menge einer Wiederablagerungs-Cellulase, die ausreichend ist, um die Variationen bezüglich der Farbdichte zu erzeugen; und
b) einer wirksamen Menge einer hinzugefügten Protease, die ausreichend ist, um die Wiederanfärbung zu verringern und den Kontrast zwischen blauen und weißen Fasern zu erhöhen;
mit der Massgabe, dass die Cellulase keine Enzympräparation von Cellulasen und Amylasen enthaltendem *Penicillium funiculosum* ist.

2. Verfahren nach Anspruch 1, wobei die wirksame Menge von Cellulase und Protease einem Verhältnis von insgesamt etwa 5 ppm an Wiederablagerungs-Cellulaseprotein:1000 ppm an insgesamt hinzugefügtem Proteaseprotein zu insgesamt 1000 ppm an Wiederablagerungs-Cellulaseprotein: 1 ppm an insgesamt hinzugefügtem Proteaseprotein entspricht.

3. Verfahren nach Anspruch 1 oder Anspruch 2, wobei die hinzugefügte Protease eine Serin- oder Metalloprotease ist.

4. Verfahren nach mindestens einem der Ansprüche 1 bis 3, wobei die Protease von *Bacillus* oder *Aspergillus* stammt.

5. Verfahren nach mindestens einem der vorhergehenden Ansprüche, weiterhin umfassend die Zusetzung eines Tensids in einer Menge, die ausreichend ist, um die Entfernung von wieder abgelagertem Indigofarbstoff weiter zu erhöhen.

6. Verfahren nach mindestens einem der vorhergehenden Ansprüche, wobei die Wiederablagerungs-Cellulase von *Trichoderma* stammt.

7. Verfahren nach mindestens einem der vorhergehenden Ansprüche, wobei die hinzugefügte Protease oder die Wiederablagerungs-Cellulase durch Gentechnologie modifiziert wurde unter Erhalt eines Stammes, welcher spezielle Komponenten der Protease oder Cellulase überexprimiert, oder dem es an spezifischen Komponenten mangelt.

8. Verfahren nach mindestens einem der vorhergehenden Ansprüche, wobei die hinzugefügte Protease oder die Wiederablagerungs-Celiulase durch proteintechnische Pozesse modifiziert wurde unter Erhalt von Protease- oder Cellulaseenzymen mit veränderten Eigenschaften, wie Enzymaktivität oder -spezifität.

9. Verfahren nach mindestens einem der vorhergehenden Ansprüche, weiterhin umfassend das Kontaktieren des Denims mit Bimssteinen.

10. Verfahren nach Anspruch 3, wobei die Serinprotease ein Subtilisin ist.

11. Verfahren nach Anspruch 1, wobei die Wiederablagerungs-Cellulase und die Protease zusammen vermischt werden vor dem Kontaktieren des Denims und die Mischung auf einer Temperatur im Bereich von etwa 30°C bis etwa 60°C während etwa 1 Stunde bis zu etwa 14 Tagen gehalten wird.

12. Verwendung einer Protease und einer Wiederablagerungs-Cellulase zur Verminderung der Wiederanfärbung, wenn die Cellulase zur Einführung lokaler Variationsbereiche bezüglich der Farbdichte in die Oberfläche von indigo-gefärbtem Denim verwendet wird, wodurch ein hoher Kontrast zwischen blauen und weißen Fasern des Denims erzeugt wird.

13. Verwendung nach Anspruch 12, wobei die Cellulase und Protease in einem Verhältnis von insgesamt etwa 5 ppm an Wiederablagerungs-Cellulaseprotein : 1000 ppm an insgesamt hinzugefügtem Proteaseprotein zu insgesamt 1000 ppm an Wiederablagerungs-Cellulaseprotein : 1 ppm an insgesamt hinzugefügtem Proteaseprotein verwendet werden.

14. Verwendung nach Anspruch 12 oder Anspruch 13, wobei die Protease eine Serin- oder Metalloprotease ist.

15. Verwendung nach mindestens einem der Ansprüche 12 bis 14, wobei die Protease von *Bacillus* oder *Aspergillus* stammt.

16. Verwendung nach mindestens einem der Ansprüche 12 bis 15, weiterhin umfassend das Kontaktieren des Denims mit einem Tensid in einer Menge, die ausreichend ist, um die Entfernung von wieder abgelagertem Indigofarbstoff weiter zu erhöhen.

17. Verwendung nach mindestens einem der Ansprüche 12 bis 16, wobei die Wiederablagerungs-Cellulase von *Trichoderma* stammt.

18. Verwendung nach mindestens einem der Ansprüche 12 bis 17, wobei die Protease oder die Wiederablagerungs- Cellulase durch Gentechnologie modifiziert wurde unter Erhalt eines Stammes, welcher spezielle Komponenten der Protease oder Cellulase überexprimiert, oder dem es an spezifischen Komponenten mangelt.

19. Verwendung nach mindestens einem der Ansprüche 12 bis 18, wobei die Protease oder die Wiederablagerungs-Cellulase durch proteintechnische Prozesse modifiziert wurde unter Erhalt von Protease- oder Cellulaseenzymen mit veränderten Eigenschaften, wie Enzymaktivität oder -spezifität.

20. Verwendung nach mindestens einem der Ansprüche 12 bis 19, weiterhin umfassend das Kontaktieren des Denims mit Bimssteinen.

21. Verwendung nach Anspruch 14, wobei die Serinprotease ein Subtilisin ist.

22. Verwendung nach mindestens einem der Ansprüche 12 bis 21, wobei die Wiederablagerungs-Cellulase und die Protease vor dem Kontaktieren des Denims zusammen vermischt werden und die Mischung auf einer Temperatur im Bereich von etwa 30°C bis etwa 60°C während etwa 1 Stunde bis zu etwa 14 Tagen gehalten wird.

## Revendications

1. Procédé pour introduire dans la surface de la matière denim teinte en indigo, des zones localisées de variation de densité de couleur et un contraste élevé entre les fibres bleues et blanches du denim, **caractérisé en ce que** ledit procédé comprend la mise en contact du denim simultanément avec :
a. une quantité effective d'une cellulase de redéposition suffisante pour produire les variations de densité de couleur ; et,
b. une quantité effective d'une protéase ajoutée suffisante pour réduire la déteinture et augmenter le contraste entre les fibres bleues et blanches ;
sous réserve que la cellulase ne soit pas une préparation enzymatique de Penicillium funiculosum contenant des cellulases et des amylases.

2. Procédé selon la revendication 1, dans lequel la quantité effective de cellulase et de protéase est un rapport compris entre environ 5 ppm de protéine de cellulase de redéposition totale : 1 000 ppm de protéine de protéase ajoutée totale, et 1 000 ppm de protéine de cellulase de redéposition totale : 1 ppm de protéine de protéase ajoutée totale.

3. Procédé selon la revendication 1 ou la revendication 2, dans lequel ladite protéase ajoutée est une protéase sérine ou une métalloprotéase.

4. Procédé selon l'une quelconque des revendications 1 à 3, dans lequel la protéase est dérivée de *Bacillus* ou *d'Aspergillus.*

5. Procédé selon l'une quelconque des revendications précédentes, comprenant, de plus, l'addition d'un agent tensioactif dans une quantité suffisante pour augmenter davantage l'enlèvement de la teinture indigo redéposée.

6. Procédé selon l'une quelconque des revendications précédentes, dans lequel la cellulase de redéposition est dérivée de *Trichoderma.*

7. Procédé selon l'une quelconque des revendications précédentes, dans lequel ladite protéase ajoutée ou ladite cellulase de redéposition a été modifiée par ingénierie génétique pour produire une souche qui surexprime des composants particuliers de la protéase ou de la cellulase ou qui est supprimée pour des composants spécifiques.

8. Procédé selon l'une quelconque des revendications précédentes, dans lequel ladite protéase ajoutée ou ladite cellulase de redéposition a été modifiée par ingénierie génétique pour produire des enzymes cellulase ou protéase ayant des propriétés modifiées, telles que l'activité ou la spécificité enzymatique.

9. Procédé selon l'une quelconque des revendications précédentes, comprenant, de plus, la mise en contact du denim avec des pierres ponce.

10. Procédé selon la revendication 3, dans lequel ladite protéase sérine est une subtilisine.

11. Procédé selon la revendication 1, dans lequel ladite cellulase de redéposition et ladite protéase sont mélangées ensemble avant la mise en contact avec ledit denim et dans lequel ledit mélange est maintenu à une température comprise entre environ 30 °C et environ 60 °C pendant environ 1 heure à environ 14 jours.

12. Utilisation d'une protéase et d'une cellulase de redéposition pour réduire la déteinture lorsque la cellulase est utilisée pour introduire des zones localisées de variation de densité de couleur dans la surface du denim teint en indigo, produisant, de ce fait, un contraste élevé entre les fibres bleues et blanches du denim.

13. Utilisation selon la revendication 12, dans laquelle la cellulase et la protéase sont utilisées dans un rapport compris entre environ 5 ppm de protéine de cellulase de redéposition totale : 1 000 ppm de protéine de protéase ajoutée totale, et 1 000 ppm de protéine de cellulase de redéposition totale : 1 ppm de protéine de protéase ajoutée totale.

14. Utilisation selon la revendication 12 ou la revendication 13, dans laquelle la protéase est une protéase sérine ou une métalloprotéase.

15. Utilisation selon l'une quelconque des revendications 12 à 14, dans laquelle la protéase est dérivée de *Bacillus* ou *d'Aspergillus.*

16. Utilisation selon l'une quelconque des revendications 12 à 15, comprenant, de plus, la mise en contact du denim avec un agent tensioactif dans une quantité suffisante pour augmenter davantage l'enlèvement de la teinture indigo redéposée.

17. Utilisation selon l'une quelconque des revendications 12 à 16, dans laquelle la cellulase de redéposition est dérivée de *Trichoderma.*

18. Utilisation selon l'une quelconque des revendications 12 à 17, dans laquelle la protéase ou la cellulase de redéposition a été modifiée par ingénierie génétique pour produire une souche qui surexprime des composants particuliers de la protéase ou de la cellulase ou qui est supprimée pour des composants spécifiques.

19. Utilisation selon l'une quelconque des revendications 12 à 18, dans laquelle la protéase ajoutée ou la cellulase de redéposition a été modifiée par ingénierie génétique pour produire des enzymes cellulase ou protéase ayant des propriétés modifiées, telles que l'activité ou la spécificité enzymatique.

20. Utilisation selon l'une quelconque des revendications 12 à 19, comprenant, de plus, la mise en contact du denim avec des pierres ponce.

21. Utilisation selon la revendication 14, dans lequel la protéase sérine est une subtilisine.

22. Utilisation selon l'une quelconque des revendications 12 à 21, dans laquelle la cellulase de redéposition et la protéase sont mélangées ensemble avant la mise en contact avec ledit denim et dans laquelle ledit mélange est maintenu à une température comprise entre environ 30 °C et environ 60 °C pendant environ 1 heure à environ 14 jours.
